# FASCICULE DE BREVET EUROPEEN

(11) **EP 0 808 127 B1**
(45) Date de publication et mention de la délivrance du brevet: **10.05.2000**
(21) Numéro de dépôt: 96902315.9
(22) Date de dépôt: 31.01.1996
(51) Int. Cl.: A61B 5/103

(54) **DISPOSITIF DE MESURE DES EFFORTS EXERCES LORS DE LA MARCHE**
VORRICHTUNG ZUR MESSUNG DER BEIM GEHEN AUFTRETENDEN KRÄFTE
DEVICE FOR MEASURING FORCES EXERTED DURING AMBULATORY EXERCISE

(30) Priorité: 08.02.1995 FR 9501692
(43) Date de publication de la demande: 26.11.1997
(73) Titulaire: CENTRE STEPHANOIS DE RECHERCHES MECANIQUES HYDROMECANIQUE ET FROTTEMENT Société anonyme, F-42166 Andrezieux Boutheon Cédex (FR)
(72) Inventeur: BELLI, Alain, F-42100 Saint-Etienne (FR); BERGER, Antoine, F-42230 Roche-la-Molière (FR)
(74) Mandataire: Thivillier, Patrick
(86) Numéro de dépôt international: FR9600162
(87) Numéro de publication internationale: WO9624286

(56) Documents cités:
- EP-A- 0 603 115
- WO-A-93/06779
- DE-U- 9 017 709
- BIOMEDIZINISCHE TECHNIK, vol. 32, no. 10, BERLIN (DE), pages 250-254, XP002003201 G. A. HORTSMANN ET AL.: "Speziallaufband für Stand- und Ganguntersuchungen in Forshung und Klinik"

## Description

L'invention se rattache au secteur technique des appareils d'analyse et de mesure à usage médical biomécanique notamment, médical et sportif.

Dans le cas d'une rééducation fonctionnelle, suite à des traumatismes quelconques, ou bien simplement dans le cas de contrôle et de test d'un individu, il est important de connaître les efforts appliqués par chacune des jambes, lors d'une marche naturelle.

On connaît des appareils permettant de mesurer les variations angulaires entre notamment le tibia et le fémur, correspondant notamment, aux mouvements de flexion et d'extension lors de la marche. Par contre, de tels appareils ne donnent aucune indication sur les forces d'appui.

Pour mesurer les forces d'appui, on connaît des systèmes mettant en oeuvre une plateforme en appui au sol, par l'intermédiaire de capteurs de mesure. La plateforme est interposée dans le trajet, de manière à obtenir l'image d'un appui au cours de la marche. Il apparait cependant qu'une telle solution n'est pas satisfaisante, étant donné que, naturellement, la personne a tendance à marquer un temps d'arrêt avant de marcher sur la plateforme, de sorte que l'appui exercé n'est pas naturel. Ce système peut être doublé pour chaque jambe. Ce système n'est pas adapté à la mesure de plusieurs pas consécutifs.

On a proposé également d'équiper des tapis roulants, pour tenter de mesurer les efforts d'appui appliqués lors du déplacement d'un individu. Ce système consiste à interposer des capteurs d'efforts entre la sole, sur laquelle glisse la bande du tapis, et le châssis. Une telle solution entraine plusieurs inconvénients :
- la mesure ne différencie pas l'appui de chaque jambe, ce qui est peu gênant pour l'analyse de la course, car il n'y a pratiquement jamais un appui simultané des deux jambes, l'appui étant essentiellement monopodal, mais ce qui est rédhibitoire pour la marche, étant donné qu'il y a systématiquement un appui simultané des deux jambes, l'appui étant du type bipodal,
- la mesure des efforts tangentiels est impossible.

Par le brevet EP-A-0603115 et la publication BIOMEDIZINISCHE TECHNIK, Volume 32, N. 10, Octobre 1987, on connaît des solutions dans lesquelles le tapis roulant est en deux parties séparées. Dans le brevet EP-A-0603115, la séparation est faite au niveau transversal, tandis que dans la publication BIOMEDIZINISCHE TECHNIK, la séparation est faite au niveau longitudinal. Cela étant, dans l'un et l'autre cas, les capteurs de mesure sont disposés entre les tapis et la sole, c'est-à-dire la partie sur laquelle glissent les tapis. Il apparait donc que ces solutions ne permettent pas de mesurer les efforts tangentiels, compte-tenu du frottement des tapis sur la sole.

L'invention s'est fixée pour but de remédier à ces inconvénients, d'une manière simple, sûre, efficace et rationnelle.

Le problème que se propose de résoudre l'invention est de proposer aux physiologistes et aux orthopédistes, une solution apte à mesurer les efforts verticaux et horizontaux, c'est-à-dire les efforts tangentiels, des appuis au sol, notamment de plusieurs pas successifs, en ) séparant avantageusement mais non limitativement les appuis exercés par la jambe droite de ceux exercés par la jambe gauche.

Pour résoudre un tel problème, il a été conçu et mis au point un dispositif de mesure des efforts exercés par chaque jambe lors de la marche notamment, qui comprend au moins un ensemble équipé d'un tapis roulant motorisé, monté dans un châssis constitué par une structure rigide recevant les différents éléments du tapis pour son entrainement, ladite structure étant portée par des capteurs de mesure de force solidaires d'une plaque commune d'appui au sol, afin de mesurer les efforts verticaux et tangentiels.

Il apparait donc que l'on mesure les efforts verticaux et tangentiels étant donné que l'on s'affranchit totalement des frottements, la partie recevant le tapis roulant étant isolée par rapport au sol.

Dans ces conditions, les forces de frottement du tapis sur son support et de tous les éléments d'entraînement du tapis, sont des forces internes qui, en aucun cas, ne sont appliquées aux capteurs d'appui portant la structure.

Pour résoudre le problème posé de mesurer les différents efforts d'appui exercés par chacune des jambes de plusieurs pas consécutifs pendant la marche, le dispositif comprend deux ensembles indépendants disposés l'un à côté de l'autre, d'une manière parallèle, pour correspondre à chacune des jambes, chacun des ensembles étant assujettis, d'une manière séparée, aux capteurs de mesure de force portés par la plaque commune d'appui au sol.

Avantageusement, chacune des structures comprend une partie supérieure sous forme d'une poutre rectiligne, agencée pour le montage d'une bande sans fin montée en combinaison avec des rouleaux d'entrainement fixés à chacune des extrémités de ladite poutre, lesdites structures étant disposées en regard l'une de l'autre, de manière à positionner les tapis de manière quasi jointive, en évitant tout contact entre les deux tapis.

Pour résoudre le problème posé de mesurer de manière simple les efforts d'appui verticaux et tangentiels et avoir accès, avec précision, à la variation rapide de ceux-ci, les capteurs peuvent être des capteurs de force à quartz aptes à mesurer les trois composantes orthogonales d'une force dynamique agissant dans une direction quelconque.

Avantageusement, les capteurs sont fixés à chacune des extrémités de chaque châssis.

L'invention est exposée, ci-après plus en détail à l'aide des dessins annexés, dans lesquels :

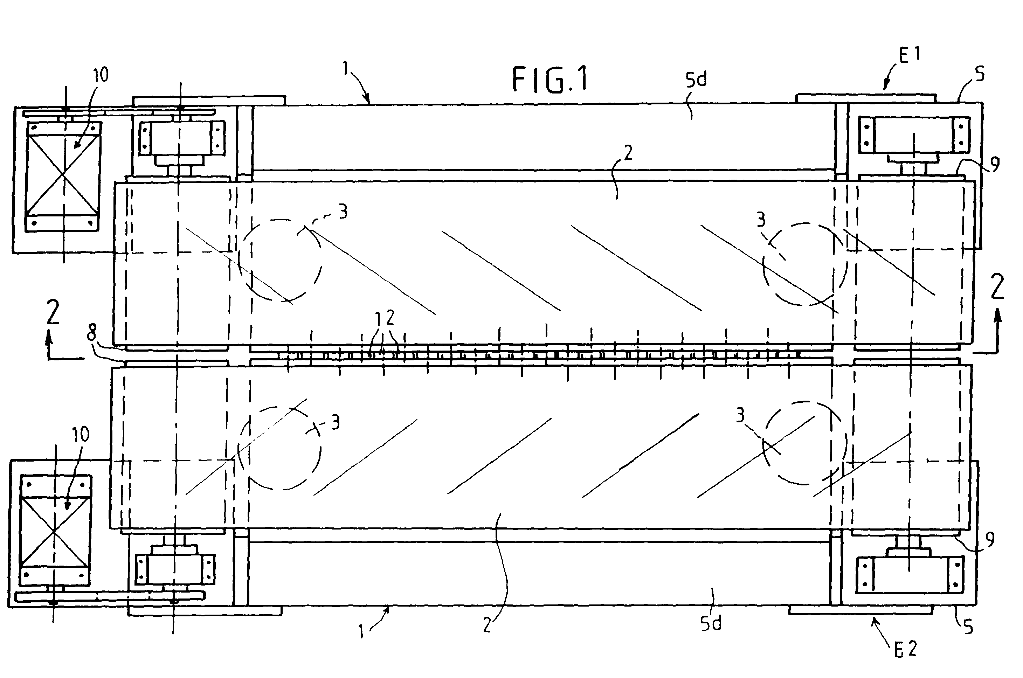

La figure 1 est une vue en plan du dispositif.

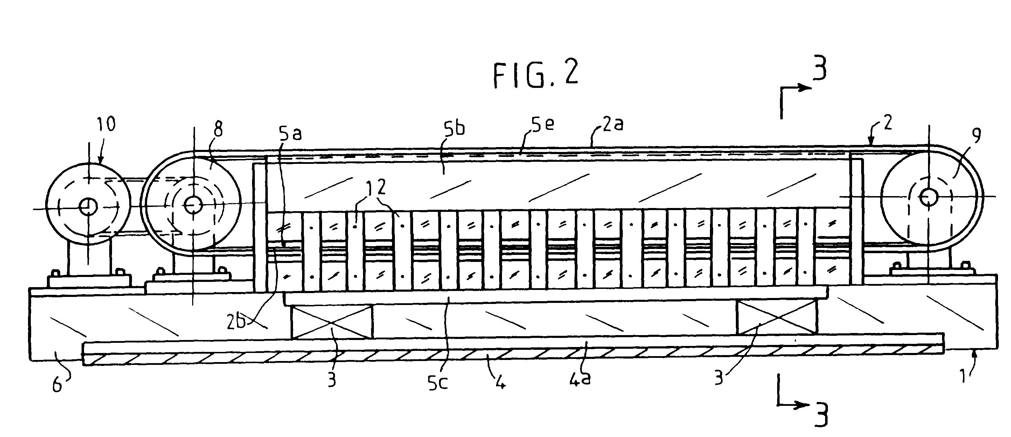

La figure 2 est une vue en coupe longitudinale considérée selon la ligne 2.2 de la figure 1.

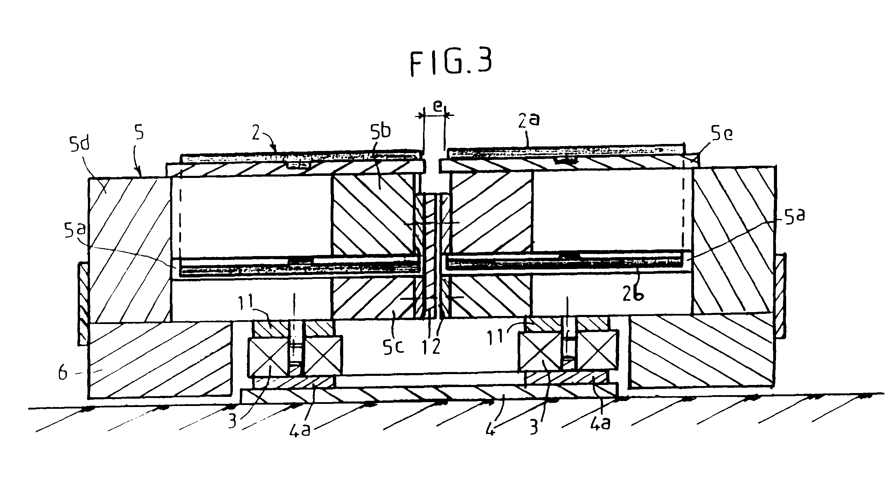

La figure 3 est une vue en coupe transversale considérée selon la ligne 3.3 de la figure 2.

Selon l'invention, le dispositif comprend deux ensembles indépendants (E1) et (E2), disposés l'un à côté de l'autre, d'une manière parallèle. Chaque ensemble (E1) et (E2) constitue un châssis support (1), conformé pour le montage d'un tapis roulant motorisé (2).

Chacun des châssis (1) est assujetti à des capteurs de mesure de force (3) portés par une plaque commune d'appui au sol (4).

Selon l'invention, chacun des châssis est conformé pour constituer une structure rigide et isolée dans l'espace, afin de pouvoir mesurer de manière efficace, les efforts verticaux et tangentiels en combinaison avec les capteurs (3), en totale indépendance des frottements. Dans l'exemple de réalisation illustré, chacune des structures comprend une partie supérieure sous forme d'une poutre rectiligne (5), présentant une fente (5a) pour le passage de la bande sans fin (2a) du tapis roulant (2).

Le brin supérieur (2b) de la bande s'appuie sur une plaque ou sole (5e) dont la surface de contact avec la bande est traitée pour améliorer le glissement.

Cette poutre (5) peut être monobloc ou composée de deux longerons parallèles (5b) (5c) délimitant la fente (5a), les longerons étant accouplés deux à deux, par un longeron latéral (5d). La poutre (5), quelle que soit sa forme de réalisation, peut également être accouplée au niveau de sa face de dessous et, du côté de son bras externe, à un autre longeron (6), dont la fonction essentielle est d'augmenter encore la rigidité de la poutre (5). En outre, ce longeron (6) déborde des faces transversales de la poutre (5) et peut présenter des semelles d'appui (7) pour le montage des rouleaux d'entrainement (8) et (9) du tapis (2).

D'une manière classique, l'un des rouleaux (8) par exemple, est motorisé. Le rouleau (8) peut intégrer directement un moteur, ou être accouplé par tout moyen connu et approprié à un moteur indépendant (10), de manière à entrainer chacun des tapis (2) dans un sens opposé à celui de la marche.

L'ensemble du châssis tel que défini repose sur la plaque support d'appui au sol (4) par l'intermédiaire des capteurs (3). Par exemple, la face de dessous de chacune des poutres (5) présente un rail rectifié (11) sur lequel est fixée l'une des extrémités des capteurs (3). L'autre extrémité des capteurs est fixée sur des rails parallèles (4a) de la plaque commune d'appui au sol (4).

Les deux châssis sont strictement identiques et sont disposés en regard l'un de l'autre de manière parallèle, en étant accouplés sur la plaque commune (4), par l'intermédiaire des capteurs (3), en laissant subsister entre eux, un espace (e) très réduit. Il en résulte que les tapis roulants (2), après montage sur les rouleaux d'entrainement correspondant (8) et (9) sont indépendants, mais sont quasiment jointifs.

Pour mesurer indépendamment les efforts d'appui et particulièrement les efforts tangentiels, il est indispensable d'entraîner chacun des tapis par un moteur indépendant.

En ce qui concerne les capteurs (3), ces derniers sont avantageusement constitués par des capteurs de force à quartz, aptes à mesurer les trois composantes orthogonales d'une force dynamique ou quasi statique, agissant dans une direction quelconque. Avantageusement, ces capteurs peuvent être du type de ceux commercialisés par la société KISTLER INSTRUMENTS.

Ces dispositions constructives permettent donc de séparer la jambe droite de la jambe gauche et par conséquent, de mesurer, indépendemment, les efforts d'appui verticaux et tangentiels correspondant à un appui bipodal exercé lors de la marche.

Bien évidement, l'ensemble du dispositif tel que décrit est asservi à tout système de traitement et d'analyse des mesures relevées.

On prévoit également, pour rigidifier l'ensemble de la poutre (5), de relier les deux longerons parallèles (5b) (5c), de barrettes verticales espacées (12). A noter que les barrettes de rigidité des poutres des deux ensembles opposés sont fixées deux à deux de manière régulièrement alternée, pour réduire l'espace entre lesdites deux poutres, après accouplement sur la plaque commune d'appui au sol en combinaison avec les capteurs

Sans pour cela sortir du cadre de l'invention, on prévoit de réaliser un châssis apte à recevoir un seul tapis roulant motorisé, et non plus des tapis indépendants correspondant à chacune des jambes. Dans ce cas, le dispositif trouve une application essentiellement pour mesurer les efforts dans le cas de la course où les appuis sont essentiellent du type monopodal.

Les avantages ressortent bien de la description, en particulier on souligne et on rappelle :
- la possibilité de mesurer de manière indépendante, les efforts verticaux et tangentiels exercés par chacune des jambes lors de la marche,
- la simplicité de réalisation,
- l'efficacité du résultat obtenu et sa très grande précision, la mesure étant complètement indépendante des frottements et des efforts d'entrainements mécaniques des tapis.

## Revendications

1. Dispositif de mesure des efforts exercés par chaque jambe lors de la marche notamment, comprenant au moins un ensemble équipé d'un tapis roulant motorisé, caractérisé en ce que l'ensemble équipé d'un tapis roulant est monté dans un châssis constitué par une structure rigide recevant les différents éléments du tapis (2) pour son entrainement, ladite structure étant portée par des capteurs de mesure de force (3) solidaires d'une plaque commune d'appui au sol (4), afin de mesurer les efforts verticaux et tangentiels.

2. Dispositif selon la revendication 1, en ce qu'il comprend deux ensembles indépendants (E1) (E2) disposés l'un à côté de l'autre, d'une manière parallèle, pour correspondre à chacune des jambes, chacun des ensembles étant assujettis, d'une manière séparée, aux capteurs de mesure de force (3) portés par la plaque commune d'appui au sol (4).

3. Dispositif selon la revendication 1, caractérisé en ce que chaque ensemble est équipé d'un tapis roulant (2) motorisé pour être entrainé linéairement dans un sens inverse à celui de la marche, chaque tapis (2) étant monté dans le châssis (1).

4. Dispositif selon la revendication 1, caractérisé en ce que chacune des structures comprend une partie supérieure sous forme d'une poutre rectiligne (5), agencée pour le montage d'une bande sans fin montée en combinaison avec des rouleaux d'entrainement (8) (9) fixés à chacune des extrémités de ladite poutre (5), lesdites structures étant disposées en regard l'une de l'autre, de manière à positionner les tapis de manière quasi jointive sans contact.

5. Dispositif selon la revendication 4, caractérisé en ce que la poutre (5) est composé de deux longerons parallèles (5b) (5c) délimitant une fente (5a) pour le passage de la bande sans fin (2a) du tapis roulant (2), lesdits longerons étant accouplés par un longeron latéral (5d), ladite poutre étant en outre, reliée à un longeron de rigidité (6), agencé pour le montage des rouleaux d'entrainement (8) et (9).

6. Dispositif selon la revendication 1, caractérisé en ce que les capteurs (3) sont des capteurs de force à quartz aptes à mesurer les trois composantes orthogonales d'une force dynamique rapidement variable, agissant dans une direction quelconque.

7. Dispositif selon la revendication 1, caractérisé en ce que les capteurs (3) sont fixés à chacune des extrémités de chaque châssis (1).

## Patentansprüche

1. Vorrichtung zur Messung von durch jedes Bein insbesondere beim Gehen ausgeübten Kräften mit mindestens einer Anordnung, die mit einem motorisierten Laufband ausgestattet ist, dadurch gekennzeichnet, daß die mit einem Laufband ausgestattete Anordnung in einem Rahmen angebracht ist, der aus einer starren Konstruktion besteht, welche die verschiedenen Elemente des Laufbands (2) für dessen Antrieb aufnimmt, wobei die Konstruktion von Kraftmeßsensoren (3) getragen wird, die mit einer gemeinsamen Bodenstützplatte (4) fest verbunden sind, um die vertikalen und tangentialen Kräfte zu messen.

2. Vorrichtung nach Anspruch 1, dadurch gekennzeichnet, daß sie zwei unabhängige Anordnungen (E1) (E2) umfaßt, die parallel nebeneinander so angeordnet sind, daß sie jeweils einem Bein zugeordnet sind, wobei jede der Anordnungen auf getrennte Weise mit den von der gemeinsamen Bodenstützplatte (4) getragenen Kraftmeßsensoren (3) verbunden ist.

3. Vorrichtung nach Anspruch 1, dadurch gekennzeichnet, daß jede Anordnung mit einem Laufband (2) versehen ist, das so motorisiert ist, daß es in einer zur Gehrichtung entgegengesetzten Richtung linear angetrieben wird, wobei jedes Band (2) in dem Rahmen (1) angebracht ist.

4. Vorrichtung nach Anspruch 1, dadurch gekennzeichnet, daß jede der Konstruktionen einen oberen Teil in Form eines geraden Trägers (5) umfaßt, der zur Befestigung eines Endlosbands angeordnet ist, das in Kombination mit jeweils an jedem der Enden des Trägers (5) befestigten Antriebsrollen (8) (9) angebracht ist, wobei die Konstruktionen einander gegenüber so angeordnet sind, daß die Bänder fast aneinander angrenzend ohne Kontakt positioniert werden.

5. Vorrichtung nach Anspruch 4, dadurch gekennzeichnet, daß der Träger (5) aus zwei parallelen Längsträgern (5b) (5c) besteht, die einen Schlitz (5a) zur Durchführung des Endlosbands (2a) des Laufbands (2) begrenzen, wobei die Längsträger durch einen Seitenlängsträger (5d) miteinander verbunden sind, wobei der Träger des weiteren mit einem Versteifungslängsträger (6) verbunden ist, der zur Befestigung der Antriebsrollen (8) und (9) angeordnet ist.

6. Vorrichtung nach Anspruch 1, dadurch gekennzeichnet, daß es sich bei den Sensoren (3) um Quarz-Kraftmeßsensoren handelt, die die drei orthogonalen Komponenten einer sich schnell ändernden dynamischen Kraft, die in einer beliebigen Richtung wirkt, messen können.

7. Vorrichtung nach Anspruch 1, dadurch gekennzeichnet, daß die Sensoren (3) an jedem der Enden jedes Rahmens (1) befestigt sind.

## Claims

1. Device for measuring the forces exerted by each leg, when walking in particular, comprising at least one assembly equipped with a motor-driven moving belt, characterised in that the assembly equipped with the moving belt is mounted in a chassis consisting of a rigid structure which accommodates the various elements of the belt (2) in order to drive it, said structure being supported by force transducers (3) attached to a common floor base plate (4) in order to measure vertical and tangential forces.

2. Device as claimed in claim 1, characterised in that it comprises two independent assemblies (E1) (E2) arranged parallel side by side, each corresponding to one leg, each of the assemblies being separately linked to force transducers (3) supported by the common floor base plate (4).

3. Device as claimed in claim 1, characterised in that each assembly is equipped with a motor-driven moving belt (2) which is moved in a linear direction in opposition to the direction of walking, each belt (2) being mounted on the chassis (1) .

4. Device as claimed in claim 1, characterised in that each of the structures comprises an upper part in the form of a straight beam (5) designed for mounting an endless belt fitted in combination with drive rollers (8) (9) fixed at each end of said beam (5), said structures being arranged opposite each other so as to position the belts so that they almost abut but do not touch each other.

5. Device as claimed in claim 4, characterised in that beam (5) consists of two parallel members (5b) (5c) which leave a gap (5a) through which the endless belt (2a) of the moving belt (2) can pass, said members being connected by a lateral member (5d), said beam also being joined to a reinforcing member (6) designed for mounting the drive rollers (8) and (9).

6. Device as claimed in claim 1, characterised in that the transducers (3) are quartz-crystal load cells capable of measuring the three orthogonal components of a rapidly variable dynamic force acting in any direction.

7. Device as claimed in claim 1, characterised in that the transducers (3) are each attached to the ends of each chassis (1).
